# EUROPEAN PATENT APPLICATION

(11) **EP 4 537 840 A1**
(43) Date of publication of application: **16.04.2025**
(21) Application number: 23203538.6
(22) Date of filing: 13.10.2023
(51) Int. Cl.: A61K 39/12, A61P 31/14, A61K 39/00

(54) **SOLUBLE HCV GLYCOROTEIN E2 AS A VACCINE AGAINST HEPATITIS C VIRUS**

(71) Applicant: Twincore, Zentrum für Experimentelle und Klinische Infektionsforschung GmbH, 30625 Hannover (DE); Universität zu Lübeck, 23562 Lübeck (DE)
(72) Inventor: Labuhn, Maurice, 30823 Garbsen (DE); Bankwitz, Dorothea, 30966 Hemmingen (DE); Krey, Thomas, 23627 Groß Sarau (DE); Pietschmann, Thomas, 30625 Hannover (DE)
(74) Representative: Moré, Solveig Helga

(57) **Abstract**

The present invention relates to the field of vaccination, in particular, of vaccination against hepatitis C virus (HCV). The present invention provides a composition comprising HCV glycoprotein E2 from strain GT4a. ED43, and optionally, further HCV glycoproteins E2 from other strains and/or an adjuvant. The glycoprotein can be soluble glycoprotein or it can be displayed on the surface of a nanoparticle. Alternatively, it provides a composition comprising a nucleic acid encoding HCV glycoprotein E2 from strain GT4a.ED43, and, optionally, from other strains. The composition is useful as a vaccine, e.g., for prophylactic vaccination against HCV. The invention also provides a method for producing said composition as well as HCV glycoprotein E2 from strain GT2b.2b5 and a nucleic acid encoding the same.

## Description

The present invention relates to the field of vaccination, in particular, of vaccination against hepatitis C virus (HCV). The present invention provides a composition comprising HCV glycoprotein E2 from strain GT4a. ED43, and optionally, further HCV glycoproteins E2 from other strains and/or an adjuvant. The glycoprotein can be soluble glycoprotein or it can be displayed on the surface of a nanoparticle. Alternatively, it provides a composition comprising a nucleic acid encoding HCV glycoprotein E2 from strain GT4a.ED43, and, optionally, from other strains. The composition is useful as a vaccine, e.g., for prophylactic vaccination against HCV. The invention also provides a method for producing said composition as well as HCV glycoprotein E2 from strain GT2b.2b5 and a nucleic acid encoding the same.

HCV has chronically infected an estimated 58 million people worldwide and is therefore a global health problem. Of those who are exposed to the virus, 50%-80% progress to a chronic infection and are at risk to develop liver cirrhosis and hepatocellular carcinoma.

The advent of highly effective direct-acting antivirals (DAAs) has revolutionised patient care. However, providing access to DAAs for all patients worldwide remains a major public health challenge. In addition, in rare cases, therapy fails due to resistance-associated variants. HCV reinfection is possible after treatment-induced cure.

Thus, a prophylactic vaccine is important for control of HCV disease burden. One approach of vaccine development capitalises on the induction of broadly neutralising antibodies (bNAbs) targeting envelope glycoproteins E1 and E2. Multiple studies support an important role of neutralising antibodies for spontaneous HCV clearance (Osburn et al., 2010; Pestka et al., 2007).

Moreover, passive immune-prophylaxis studies in animals confirm the importance of antibodies in protecting from HCV infection (Law et al., 2008; Vanwolleghem et al., 2008).

However, due to the extraordinary diversity and flexibility of HCV as well as different immune evasion mechanisms of HCV, HCV is a difficult target for development of a vaccine. Further, there are limitations in testing vaccine candidates, as there is no robust animal model for direct protection from HCV infection (Bankwitz et al., 2022).

HCV is highly variable and viral isolates are classified into eight genotypes (GTs) and multiple subtypes. The genetic diversity even surpasses that of HIV-1 (Smith et al., 2014).

The envelope proteins are targets of neutralising antibodies and represent the most variable proteins of the virus. Hypervariable regions (HVR), e.g., HVR1 in E2 protein, are highly immunogenic and may lead to induction of antibodies that are not protective. HVR1 may even be deleted without the virus loosing infectivity (Bankwitz et al., 2010). For successful vaccine design, it is crucial to elicit cross-protective antibodies against diverse HCV variants.

However, lack of a robust animal model and use of different experimental systems for quantification of antibody responses complicates comparison of vaccine efficacy. Typically, either infection assays based on retroviral HCV pseudoparticles (HCVpp) or cell culture-derived HCV particles (HCVcc) are used to quantify antibody efficacy. In recent years, several panels of HCVpp or HCVcc were developed (Kinchen et al., 2018). In part, these panels include a large number of different E1-E2 proteins (Tarr et al., 2011). However, in some cases, these panels only encompass GT1-derived glycoproteins (Osburn et al., 2014; Wasilewski et al., 2016). It is unclear if these GT-selective panels adequately report the entire functional diversity of globally sampled HCV, including GT 1 to 7 strains. In other cases, viruses with E1-E2 genes carrying cell culture-adaptive changes, which may influence virus antibody neutralisation, are included (Gottwein et al., 2009). Moreover, there are well-documented functional differences between cell entry of HCVpp and HCVcc, including dependence on entry factors and susceptibility to membrane fusion inhibitors. Although there are studies attesting a good congruence between HCVpp and HCVcc neutralisation, HCVpp tend to be more neutralisation sensitive than HCVcc (Urbanowicz et al., 2015; Wasilewski et al., 2016), and the above-mentioned differences may preclude assessment of important determinants and features of the virus-antibody interplay.

Therefore, a reference panel of viruses for rigorous and balanced quantification of breadth and potency of HCV-specific antibodies across the diversity of globally sampled HCV strains was established (Bankwitz et al., 2021). For this, the neutralisation potency of polyclonal immunoglobulins from patients infected with HCV genotype (GT) 1-6 was profiled across 13 HCV strains representing five viral GTs. Using metric multidimensional scaling, HCV neutralisation was plotted onto neutralisation maps. K-means clustering was employed to guide virus clustering and selecting representative strains. Viruses mapped to six distinct neutralisation clusters, in part composed of viruses from different GTs. Viruses differed greatly in neutralisation sensitivity, with GT2a.J6 being most resistant and GT5a.SA13 being most sensitive. There was no correlation between viral neutralisation and genetic distance, indicating functional neutralisation clustering differs from sequence-based clustering. Calibrating reference viruses representing these clusters against purified antibodies from 496 patients infected by GT1 to GT6 viruses identified individuals with extraordinary potent and broadly neutralising antibodies. It revealed comparable antibody cross-neutralisation and diversity between specimens from diverse viral GTs, confirming well-balanced reporting of HCV cross-neutralisation across highly diverse human samples.

Virus strains from this panel were e.g. used to identify HCV infected subjects with very potent antibody responses, and the most effective antibodies were cloned and analysed (Weber et al., 2022). The human antibodies created by this procedure are among the most powerful known today.

Previous approaches to vaccination are e.g., described in Bankwitz et al., 2022. One candidate vaccine tested in humans was based on viral vectors, ChAd3 and MVA, each encoding the HCV non-structural proteins NS3, NS4A, NS4B, NS5A and inactivated NS5B (NSmut) used in a heterologous prime boost strategy. Although CD4 and CD8 T cell responses were induced, there was no significant difference in incidence of chronic HCV infection (Capone et al., 2006; Swadling et al., 2014).

In contrast, other approaches focused on the induction of antibodies, e.g., inactivated HCV particles obtained from cell culture, which can induce protective antibodies (Akazawa et al., 2013; Gottwein and Bukh, 2013; Yokokawa et al., 2018). These inactivated HCV particles are however difficult to concentrate and purify (Nestola et al., 2015; Wolf and Reichl, 2011).

Vesicular stomatitis virus (VSV)-based vaccine vectors were employed as vectors for generation of HCV virus-like particles (VLP), which induced humoral and cellular immune responses (Ezelle et al., 2002). Similar VSV-based vectors have been successfully used for protection against SARS-CoV-2 pathogenesis in mice (Case et al., 2020) and for an ebola vaccine, VSV-EBOV (Jones et al., 2005).

Tests of VLP encoding E1 and E2 produced in the insect cell line Sf9 by means of a baculovirus however did not leed to induction of neutralising antibodies in 3 out of 4 chimpanzees (Elmowalid et al., 2007). Other VLP were targeted to dendritic cells with a lipopeptide or a mixture of different HCV genotypes was used as quadrivalent vaccine candidates (Christiansen et al., 2018). HCV glycoproteins were also included in chimeric HBV-HCV particles (Beaumont et al., 2013).

Recent approaches are based on recombinantly produced HCV proteins, e.g., HCV-E1E2 (rE1E2), which was successfully tested in chimpanzees and a clinical phase I study (Choo et al., 1994; Frey et al., 2010; Ray et al., 2010), or a secreted form of the E1E2 heterodimer (Wang et al., 2022). Most approaches, however, do not take the genetic variability of HCV into account. Moreover, broad comparison of the immunogenicity of genetically and functionally diverse HCV immunogens is lacking.

In light of this, the inventors addressed the problem of providing a vaccine suitable for use in prophylactic vaccination of humans against HCV overcoming problems arising with prior art vaccine candidates, e.g., a vaccine suitable for protecting against a plurality, preferably, all HCV strains. This problem is solved by the present invention, in particular, the subject matter of the claims.

The invention provides a composition comprising glycoprotein E2 from HCV strain GT4a.ED43, wherein the glycoprotein is
i) a soluble glycoprotein not comprising a transmembrane region, and/or
ii) displayed on the surface of a nanoparticle.

Said composition may optionally comprise an adjuvant. In particular, if the composition comprises the soluble glycoprotein, it preferably comprises an adjuvant. A composition comprising a nanoparticle with the glycoprotein E2 from HCV strain GT4a.ED43 displayed on the surface of the nanoparticle may also optionally comprise an adjuvant.

The E2 glycoprotein of the composition of the invention is derived from HCV strain GT4a.ED43. As further shown herein, the inventors were able to demonstrate that in the context of the invention, the glycoprotein E2 from HCV strain GT4a.ED43, e.g. genotype GT4a (Genebank accession No. GU814265.1, https://www.ncbi.nlm.nih.qov/nuccore/GU814265.1), present in soluble form or on the surface of a nanoparticle induces antibodies with particularly good neutralising characteristics and a high cross-reactivity between strains.

HCV is not only genetically highly diverse, also viral strains exhibit remarkable functional diversity. Considering HCV envelope proteins E1/E2 (the target of neutralizing antibodies), which are critical for cell surface attachment and cell entry of virus particles, several types of functional differences have been described. First, it has been noted that viral E1-E2 proteins differ in usage of cell entry factors (Claudin 1 only or Claudin 1, 6, 9) (Haid et al., 2014; Zheng et al., 2007). This also relates to OCLN usage (Sourisseau et al., 2013). Second, HCV E1-E2 proteins differ widely in their susceptibility to neutralizing antibodies (Bankwitz et al., 2021). Finally, HCV E1-E2 protein differ in their requirement for low pH-triggered membrane fusion. While some strains fuse already in presence of relatively neutral pH, others require a much lower pH to trigger membrane fusion (Banda et al., 2019). Collectively, these results document wide functional differences between HCV E1-E2 proteins. Given that E1-E2 are metastable proteins, which upon receiving certain cues (receptor interactions, low pH environment) induce membrane fusion, it is reasonable to assume that the above-mentioned differences may be the consequence of varying degrees of metastability between these different E1-E2 proteins. Considering recent observations (SARS-CoV-2 and RSV) that stabilization of the metastable pre-fusion conformation of the respective spike proteins enhances their potential to induce neutralizing antibodies, it is possibe that different HCV E2 proteins may naturally have differentially stable pre-fusion conformations, which may make them better (or worse) vaccine antigens. Following these considerations, the inventors postulated that different E2 proteins may naturally have different capacities to induce neutralizing antibodies. We assumed that testing a wide range of antigens representing highly diverse functional and genetic properties should reveal a much improved antigen as blueprint for vaccine (or the development of a further improved candiate by additional structure/computational optimization).

In particular, as shown in detail below, there was a huge variability between E2 glycoproteins of the different strains tested with regard to their capability of inducing neutralising antibodies that could, e.g., reduce infectivity. With E2 glycoprotein from some strains, there was no reduction, but a reduction of more than factor 2 was seen with glycoprotein from HCV strain GT4a.ED43. Of note the induced antibodies were most effective in neutralising all tested reference viruses.

Protein vaccines have been used for a long time now, and are therefore very accepted in the population. Further, by choosing different adjuvants, the immunity that is induced can be regulated.

The glycoprotein E2 in a preferred embodiment is soluble E2 glycoprotein that can be expressed, e.g., as a secreted, truncated protein, purified and mixed with adjuvant for production of a vaccine. The protein can e.g., be expressed in stable transfectant of an insect cell line such as Drosophila S2 cells under the control of an MT promotor. It may also be expressed in mammalian cells, such as CHO, HEK or BHK cells. The soluble glycoprotein typically comprises the extracellular portion of the HCV glycoprotein, and it does not comprise the transmembrane or intracellular domain (in particular, the 31 C-terminal amino acids of wildtype full-length E2 glycoprotein of said strain). It may comprise HVR1 (Hypervariable region 1), but this is not required.

Soluble means in this context that the glycoprotein can be purified and stored in a pharmaceutically acceptable salt solution (e.g., 0.2 M NaCl) or buffer (e.g. Phosphate buffered saline, PBS at pH 7.2-7.4) without formation of problematic amounts of aggregates. For example, there may be less than 20% (of the soluble glycoproteins), preferably, less than 10% or less than 5% in high molecular weight aggregates. The percentage of high molecular weight aggregates can be determined, e.g., by Size Exclusion Chromatography (SEC) analysis.

The glycoprotein E2 from the specified strain can also be displayed on the surface of a nanoparticle, i.e., it can be linked to a nanoparticle or form part of it. Typically, it is covalently linked. The nanoparticle can be, e.g., a lipid nanoparticle or a self-assembling nanoparticle formed by proteins only. For example, the glycoprotein E2 can be expressed as a protein comprising a transmembrane region, which can be inserted in a lipid nanoparticle. It can also be linked to a protein-based nanoparticle, or multiple E2 proteins can form nanoparticles. The nanoparticle preferably is not a viral particle or a virus-like particle. The nanoparticles can be soluble or dispersible in a pharmaceutically acceptable salt solution (e.g., 0.2 M NaCl) or buffer (e.g. Phosphate buffered saline, PBS at pH 7.2-7.4). The glycoprotein is displayed on the surface of the nanoparticle, i.e., it is accessible to antibodies.

Nanoparticles, e.g., virus-like particles or nanocages linked to the E2 glycoproteins, have been shown to be efficient in vaccination, and they can also be employed for vaccination using the E2 protein derived from HCV strain GT4a.ED43, which has been shown by the inventors to induce particularly good cross-neutralizing antibodies. The general structure of the nanoparticles can be as disclosed in the state of the art, e.g., the publications described herein (e.g., Marcandalli et al., Cell, 2019).

Such nanoparticles can either display ED43 E2 protein only (i.e., not comprise E2 glycoprotein from other strains), in particular, as disclosed herein, or different combinations of HCV envelope proteins (e.g., 2, 3, 4, 5, 6, 7 or 8 different E2 proteins comprising ED43 E2 protein). Such combinations may be, in particular, E2 glycoprotein from HCV strain GT2b.J8 in addition to E2 glycoprotein from HCV strain GT4a.ED43, or an advantageous three strain combination as disclosed herein comprising HCV glycoprotein E2 from HCV strains GT4a.ED43, GT2b.2b5 and GT2b.J8 or HCV glycoprotein E2 from HCV strains GT4a.ED43, GT1a.H77 and GT2b.J8).

For example, homotypic or mosaic nanoparticles can be prepared with the approach shown by Cohen et al. (2022) based on the presently disclosed E2 protein and combinations of E2 proteins. Said approach was shown to lead to better production of neutralising antibodies.

The proteins of the invention can also be used in a nanocage system, e.g., the mi3 nanocage such as disclosed by Hills et al., (2023), in which up to four different envelope proteins can be coupled, e.g., the E2 glycoproteins from HCV strains GT4a.ED43 and GT2b.J8, or from HCV strains GT4a.ED43, GT2b.2b5 and GT2b.J8 or from HCV strains GT4a.ED43, GT1a.H77 and GT2b.J8, or from HCV strains GT4a.ED43, GT1a.H77, GT2b.2b5 and GT2b.J8.

Also, based on the approach described by Hoffman et al (2023), enveloped virus-like particules presenting the extracellular part of the ED43 E2 protein that assemble due to the addition of am EABR (ESCRT- and ALIX-binding region) motif to their cytoplasmic tail can be employed for vaccination. These can be used either directly, or as disclosed in the paper, using mRNA-LNP encoding such a modified E2 protein of the invention. Optionally, different combinations of HCV envelope proteins, e.g., as disclosed herein, can be assembled to such virus-like particles together. For this, the E2 protein is assembled with the accessory sequences disclosed by Hoffman et al.. Accordingly, the E2 protein comprises a transmembrane domain, such that it is presented in the virus-like particles in a membrane-bound form. Different transmembrane regions may be used to this end, e.g., the transmembrane region of MHC class I or other proteins. mRNAs encoding different HCV E2 proteins can be comprised in nanoparticles, e.g., lipid nanoparticles as disclosed in Hofmann et al.

Alternatively, nanoparticles as disclosed by Sliepen et al., 2022, but displaying the E2 glycoprotein from HCV strain GT2b.J8 can be used. Optionally, these nanoparticles may also comprise E2 glycoprotein from other strains, e.g., 1, 2, 3, 4, 5 or 6 other strains, in particular, the E2 glycoproteins from HCV strains GT4a.ED43 and GT2b.J8, or from HCV strains GT4a.ED43, GT2b.2b5 and GT2b.J8 or from HCV strains GT4a.ED43, GT1a.H77 and GT2b.J8, or from HCV strains GT4a.ED43, GT1a.H77, GT2b.2b5 and GT2b.J8.

The glycoprotein E2 from HCV strain GT4a.ED43 may have at least 80% sequence identity to SEQ ID NO: 1. SEQ ID NO: 1 is the sequence of the extracellular part of HCV strain GT4a.ED43 E2 glycoprotein including the hypervariable region (HVR1) encompassing the first 27 amino acid residues at the N-terminus. For sequence comparison, the sequence identity in the HVR1 of the E2 protein (E2) is ignored. The glycoprotein E2 may, preferably, also have at least 90% amino acid identity to SEQ ID NO: 1, optionally, at least 95% or at least 99% amino acid sequence identity to SEQ ID NO: 1. Preferably, the sequence of the glycoprotein E2 comprises SEQ ID NO: 1. It may also consist of one of said sequences, preferably, consist of SEQ ID NO: 1. The sequence identity of different domains or regions of said proteins may vary, in particular, the sequence identity in the hypervariable regions may be as low as 0%, e.g., 10-100%, 20-90%, 30-80%, 40-70% or 50-60%, or said region or regions may be partly or completely missing (e.g., 10%-100%, 20-90%, 30-80%, 40-90% or 50-80% thereof). The glycoprotein also belongs to the same biotype or cluster as GT4a.ED43.

The glycoprotein E2 of the composition of the invention can be a variant of the glycoprotein from wild-type GT4a.ED43, e.g., a variant modified in 1-10 or 2-5 amino acid positions (by substitution, deletion or insertion, preferably, by substitution) to further improve the immunogenic properties of the protein. Mutations that modify the overall protein stability and/or the stability of certain subdomains that modify the exposure of specific epitopes or that stabilize a putative pre-fusion confirmation or improve the capacity of the protein to activate specific germline B cell receptors may be particularly advantageous.

In an alternative embodiment of the invention, the glycoprotein E2 is not administered as a protein, but in the form of a nucleic acid for expression thereof. Therefore, the invention also provides a composition comprising a nucleic acid encoding the glycoprotein E2 from HCV strain GT4a.ED43, as described herein, wherein the nucleic acid is
a) RNA, optionally, mRNA formulated in a lipid nanoparticle, or
b) a non-HCV viral nucleic acid, optionally, comprised in a viral vecor selected from the group comprising a VSV vector, a rLCMV vector, an adenovirus vector, an MVA vector and an RNA replicon vector, or
c) DNA, wherein the nucleaic acid encoding the E2 glycoprotein preferably is operatively linked to a heterologous promotor capable of mediating expression in human cells.

As known in the art RNA may be used for vaccination, e.g., in the form of mRNA. The mRNA is typically modified to improve stability. RNA may be formulated in a lipid nanoparticle for vaccination, e.g., as disclosed by Hoffmann et al., 2023.

The nucleic acid may also be a non-HCV viral nucleic acid, e.g., a viral vecor selected from the group comprising a VSV (Vesicular Stomatitis) vector, a rLCMV (replication-deficient lymphocytic choriomeningitis virus) vector, an adenovirus vector, an MVA (Modified Vaccinia Virus Ankara) vector and an RNA replicon vector, e.g., based on the alphavirus replicase genes of Venezuelan equine encephalitis virus (VEE), Sindbis virus (SINV) or Semliki forest virus (SFV). Suitable viral vectors are known in the art, e.g., adenoviral vectors reviewed in Mendonca et al., npj Vaccines 6, Art. 97 (2021) may be used. In the context of a viral nucleic acid, the nucleic acid encoding the soluble E2 glycoprotein preferably is operably linked to a promotor, e.g., the T7 promotor. The promotor preferably is a heterologous promotor, i.e., it is not linked to a nucleic acid encoding E2 glycoprotein in HCV.

The nucleic acid may also be DNA. The nucleaic acid encoding the E2 glycoprotein preferably is operatively linked to a heterologous promotor capable of mediating expression in human cells, e.g., the T7 promotor.

It has been found that for administration of vaccines in nucleic acid form, co-administration of an additional adjuvant is not required. Without intending to be bound by the theory, it is believed that, for example, mRNA or viral nucleic acids can by themselves, or through the infection process, have an adjuvant effect, i.e., sufficiently stimulate the immune system, e.g., through Toll-like receptors (TLR) that may react to CpG nucleotides, mRNA structures or other danger signals provided by cells infected by viral nucleic acids. Nucleic acids may also be cheaper to prepare, and/or they may be prepared more quickly in response to demand. For nucleic acid vaccines, preparation processes can be standardized regardless of the sequence of the encoded protein. On the other hand, as mentioned above, protein vaccines may be more easily accepted by the population, and there is a long experience in their preparation. They may also be easierto store without degradation. Typically, viral nucleic acids, in particular, DNA viral nucleic acids, are more stable than RNA nucleic acids.

Generally, nucleic acids may be codon-optimized for expression, preferably, for expression in human cells.

In the composition of the invention, the glycoprotein E2 from HCV strain GT4a.ED43 (or, in short, ED43 E2 protein) or the nucleic acid encoding it can be the only active ingredient. Alternatively, the composition of the invention may further comprise a HCV E1 protein if the composition comprises the E2 protein, or a nucleic acid encoding a HCV E1 protein if the composition comprises a nucleic acid encoding the E2 protein. Said nucleic acid may be an additional RNA, e.g., mRNA comprised in the composition, or the E1 and E2 protein may be encoded by the same viral nucleic acid. Preferably the E1 and the E2 protein are from the same HCV strain. If the E2 glycoprotein is soluble, the E2 protein is typically also soluble. Soluble E1 protein typically consist of the extracellular domain of E1. If the E2 protein is displayed on the surface of a nanoparticle, so is the E1 protein.

The composition of the invention may further comprise at least one other HCV protein if the composition comprises the E2 protein, or another nucleic acid encoding a HCV protein if the composition comprises a nucleic acid encoding the E2 protein. Said at least one further HCV protein may be selected from the group consisting of P7 (assembly factor), NS2, NS3, NS4A, NS4B, NS5A and NS5B. Alternatively, or additionally, peptides from at least one of said proteins may be comprised or encoded, e.g., comprising at least one, preferably, at least 2, at least 5, at least 10 or at least 20 HCV T cell epitopes, such as T cell epitopes that can be recognized in the context of a plurality of HLA alleles, e.g. HLA alleles that are (together represented in at least 20%, at least 25%, at least 40% or at least 50% of the population and/or that are conserved between different HCV strains. Preferably, a combination of T cell epitopes is selected such that at least 80%, preferably, at least 90% of human individuals can present at least one of said T cell epitope on their HLA. T cell epitopes may be MHCl-restricted or MHCII-restricted T cell epitopes. Peptides comprising T cell epitopes from different HCV strains and/or from different proteins may be encoded in a separate protein or in a fusion protein with the E2 glycoprotein, e.g., soluble E2 glycoprotein. This may further enhance the immune response and enable generation of additional antibodies to these proteins and/or T cell responses. The amount of additional proteins and/or peptides encoded is limited by the maximal size of the vector, if the nucleic acid is a viral vector. Preferably, the maximal size of the vector is 16-18 kb. In one embodiment, the viral nucleic acid encodes, e.g., full length HCV core protein, E1, the E2 glycoprotein as defined herein (e.g., soluble E2 glycoprotein), P7, and NS2. Other HCV proteins can also be selected, but at least one HCV protein must be missing, thus avoiding the functional full HCV genome.

Non E2 HCV protein(s) may have or not have a modification allowing for membrane-binding, e.g., on cellular membranes. In one embodiment, the soluble glycoprotein of the invention is thus used for vaccination in combination with a membrane-bound protein expressed by a cell. The protein may comprise heterologous signal peptides, e.g., to optimize expression. It may also lack such signal proteins.

The inventors have further found that combinations of E2 glycoproteins according to the invention from certain strains are particularly good at inducing neutralising antibodies against a broad variety of strains. In particular, different combinations of E2 from three strains were tested, and specific combinations found to be of comparative efficiency as, otherwise, only 6-strain combinations, which are significantly more complicated to produce. Interestingly, both particularly advantageous 3-strain combinations comprise E2 glycoprotein from HCV strain GT2b.J8 in addition to E2 glycoprotein from HCV strain GT4a.ED43, as defined herein. Therefore, the invention also provides a composition of the invention, further comprising HCV glycoprotein E2 from HCV strain GT2b.J8 or a nucleic acid encoding said HCV glycoprotein E2, wherein the nucleic acid is RNA or a non-HCV viral nucleic acid. Said glycoprotein E2 from HCV strain GT2b.J8 may have at least 90% amino acid identity to SEQ ID NO: 2, preferably, at least 95% or at least 99% sequence identity, wherein, optionally, the sequence of the glycoprotein E2 from HCV strain GT2b.J8 comprises SEQ ID NO: 2. It may also consist of one of these sequences. As defined above, the HVR1 region is not included in the sequence comparison. It may or may not be comprised in the E2 glycoprotein. The glycoprotein also belongs to the same biotype or cluster as GT2b.J8. Both E2 glycoproteins may be soluble. Alternatively, both may be displayed on the surface of a nanoparticle.

As shown below (e.g., with the Top 3# Pool consisting of: ED43 / 2b5 /J8), particularly advantageous results were obtained with the composition of the invention, further comprising HCV glycoprotein E2 from HCV strains GT2b.2b5 and GT2b.J8, or a nucleic acid encoding said HCV glycoprotein E2, wherein the nucleic acid is RNA or a non-HCV viral nucleic acid. Said glycoprotein E2 from HCV strain GT2b.2b5 has at least 90% amino acid identity to SEQ ID NO: 3, preferably, at least 95% sequence identity, wherein, optionally, the sequence of the glycoprotein E2 from HCV strain GT2b.2b5 comprises SEQ ID NO: 3. It may also consist of one of these sequences. As defined above, the HVR1 region is not included in the sequence comparison. It may or may not be comprised in the E2 glycoprotein. GT2b.J8. The glycoprotein also belongs to the same biotype or cluster as GT2b.2b5. All E2 glycoproteins may be soluble. Alternatively, all may be displayed on the surface of a nanoparticle.

As shown below, further particularly advantageous results were obtained with the composition of the invention, further comprising HCV glycoprotein E2 from HCV strains GT1a.H77 and GT2b.J8, or a nucleic acid encoding said HCV glycoprotein E2, wherein the nucleic acid is RNA or a non-HCV viral nucleic acid. Said glycoprotein E2 from HCV strain GT1a.H77 has at least 90% amino acid identity to SEQ ID NO: 4, preferably, at least 95% sequence identity, wherein, optionally, the sequence of the glycoprotein E2 from HCV strain GT1a.H77 comprises SEQ ID NO: 4. It may also consist of one of these sequences. As defined above, the HVR2 region is not included in the sequence comparison. It may or may not be ccomprised in the E2 glycoprotein. The glycoprotein also belongs to the same biotype or cluster as GT1a.H77. All E2 glycoproteins may be soluble. Alternatively, all may be displayed on the surface of a nanoparticle.

For all combinations of soluble E2 glycoprotein from different strains, or with different proteins, it is preferred that the form of administration is the same, e.g., all E2 glycoproteins, and, if present, other proteins, are administered in the form, or all E2 glycoproteins, and, if present, other proteins, are provided in nucleic acid form, in particular, of soluble protein, or in the form of nanoparticles displaying all proteins, or all in the form of RNA, such as mRNA, or all in the form of a viral nucleic acid, typically, all encoded by the same viral nucleic acid.

If E2 proteins from defined strains (or nucleic acids encoding them) are present in the composition, in one embodiment, no further E2 glycoproteins (or nucleic acids encoding them) are present in the compositions. Alternatively, further E2 glycoproteins (or nucleic acids encoding them) may be present. Optionally, the composition does not comprise E2 glycoproteins from too many or from all HCV strains to allow for efficient production.

The composition of the invention typically comprises a suitable solvent such as a buffer and/or other excipient, e.g., a bulking agent such as trehalose and/or salts (e.g., NaCl, Tris, citrate or phosphate). The composition can be a dry, e.g., a lyophilised composition. Such dry compositions may be advantageous for storage, e.g., at up to 40°C. For administration, the composition typically is a solution comprising a buffer, i.e., a dry composition may be reconstituted before administration.

If the E2 glycoprotein is a soluble protein, the composition advantageously comprises an adjuvant to improve the immune response to it. Compositions with nanoparticles may also optionally comprise an adjuvant, but they are often also immunogenic on their own. The adjuvant may be, e.g., potassium alum, aluminium hydroxide, aluminium phosphate, calcium phosphate hydroxide, paraffin oil, Adjuvant 65 (based on peanut oil), killed bacteria of the species Bordatella pertussis, Mycobacterium bois or toxoids, e.g., pertussis toxoid, plant saponins, e.g., from Quillaha (Quil A, QS-21), soybean or Polygala senega, cytokines such as IL-1, IL-2 or IL-12, toll-like receptor agonists such as CpG or Freund's incomplete adjuvant, Monophosphoryl lipid A (MPL, e.g., in the form of AS04^{®} (comprising aluminum hydroxide and MPL) or squalene. The adjuvant may also be a combination of above-mentioned examples. It may be delivered, e.g., via nanoparticle-based delivery technologies. For administration to a human, the adjuvant should be admitted for human use. Preferably, the adjuvant comprises a squalen-based oil in water nano-emulsion selected from the group comprising AddaVax^{®}, AS03^{®} (comprising squalene, DL-alpha-tocopherol and polysorbate 80), and MF59^{®} (comprising squalene, polysorbat 80 (Tween 80), trioleate 85), preferably, AddaVax or MF59^{®}.

The composition may be a pharmaceutical composition, in particular, a vaccine. It should be suitable for administration to a human, e.g., it should be free of bacteria and otherviruses, and non-toxic. Thus, the invention provides a vaccine comprising the composition of the invention. Optionally, the vaccine is a combination vaccine, i.e., it may be combined with a vaccine against another infectious agent, e.g., hepatitis B, hepatitis A, hepatitis E, pertussis, diphteria, tetanus, poliomyelitis, haemophilus B, measles, influenza, SARS-CoV-2, FSME, or HPV.

Said vaccine typically is for use in prophylactic vaccination of a subject. The subject may be a human subject or a mouse subject, preferably, a human subject. Optionally, it is a subject that has successfully been treated for a chronic HCV infection. Unfortunately, therapy of a chronic HCV infection does not protect from reinfections, and the risk is high. In this case, the vaccination of the present invention reduces the risk of reinfection or prevents reinfection. However, the subject may also never have been infected with HCV. The subject may also be, e.g., another subject at risk for a HCV infection, e.g., a user of injectable drugs, a man having sex with men, a person exposed to an increased risk of nosocomial infection or medical personnel. Therapeutic vaccination can also be envisioned.

The invention also provides a syringe comprising the vaccine of the invention, e.g., in dry form or in solution.

Advantageously, the vaccine of the present invention induces antibodies that can endow a subject with protection from a broad variety of HCV virus strains, e.g., strains belonging to the HCV clusters GT3a.S53, GT2r.2r, GT2b.2b4, GT1b.J4, GT5a.SA13, and GT2a.J6.

The vaccine of the present invention may be administered in a prime boost regimen, wherein it is used for priming and/or for boosting an immune response to HCV, preferably, for both. In case the composition comprises a viral nucleic acid encoding the E2 glycoprotein, it is preferably not based in the same virus or on the same virus strain for both boosting and priming, but different constructs should be chosen to prevent immunity to the vector.

The present invention also provides a method for producing the composition of the invention, the vaccine of the invention or the vaccine for use of the invention, comprising
a) culturing a cell expressing the gylcoprotein E2, and
b) isolating said glycoprotein, and
c) optionally, formulating said glycoprotein in a formulation suitable for storage and/or administration as a vaccine,

Further disclosed is a method for producing the composition of the invention, the vaccine of the invention or the vaccine for use of the invention, wherein the nucleic acid is an mRNA, the method comprising
i) preparing said nuleic acid, optionally, comprising culturing a cell expressing the nucleic acid, and isolating said nucleic acid, and
ii) packaging said nucleic acid in a lipid nanoparticle and
iii) optionally, formulating said lipid nanoparticle in a formulation suitable for storage and/or administration as a vaccine.

The nucleic acid can also be produced synthetically.

Further disclosed is a method for producing the composition of the invention, the vaccine of the invention or the vaccine for use of the invention, wherein the nucleic acid is a viral nucleic acid, the method comprising
a) comprising culturing a cell comprising the nucleic acid, and
b) packaging said nucleic acid in the viral vector and
c) optionally, formulating said lipid vector in a formulation suitable for storage and/or administration as a vaccine.

The present invention is further illustrated in the following examples with reference to the accompanying figures and sequences, nevertheless, without being limited thereto. For the purposes of the present invention, all references as cited herein are incorporated by reference in their entirety.

### Legends

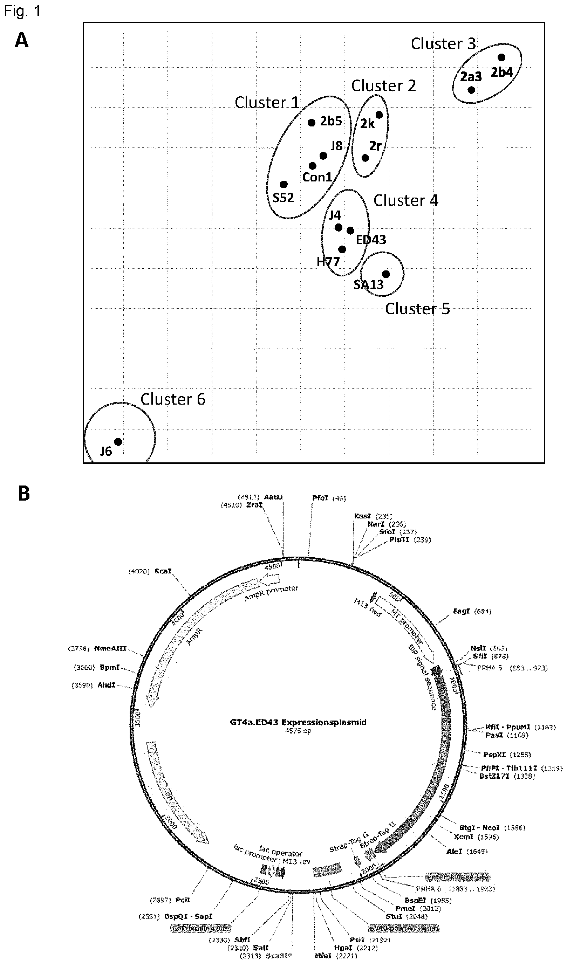

**Fig. 1****: HCV reporter-virus biotype clustering and the HCV-E1/E2-GT4a.ED43 construct.** (A) Depiction of 13 HCV reference viruses tested for neutralizability by antibodies isolated from patients. A so-called clustering was able to divide the reference viruses into 6 neutralisation clusters based on their neutralisation phenotype (Bankwitz et al., 2021). Each cluster is represented by a specific biotype (label in figure). The subdivision of these 13 reference viruses or the 6 representative biotypes forms the basis of all further approaches (Figure from: Bankwitz et al., 2021) (B) Exemplary representation of one of the generated HCV-E2 expression constructs for production of the used proteins. These were produced in insect cell lines under the control of an MT promotor.

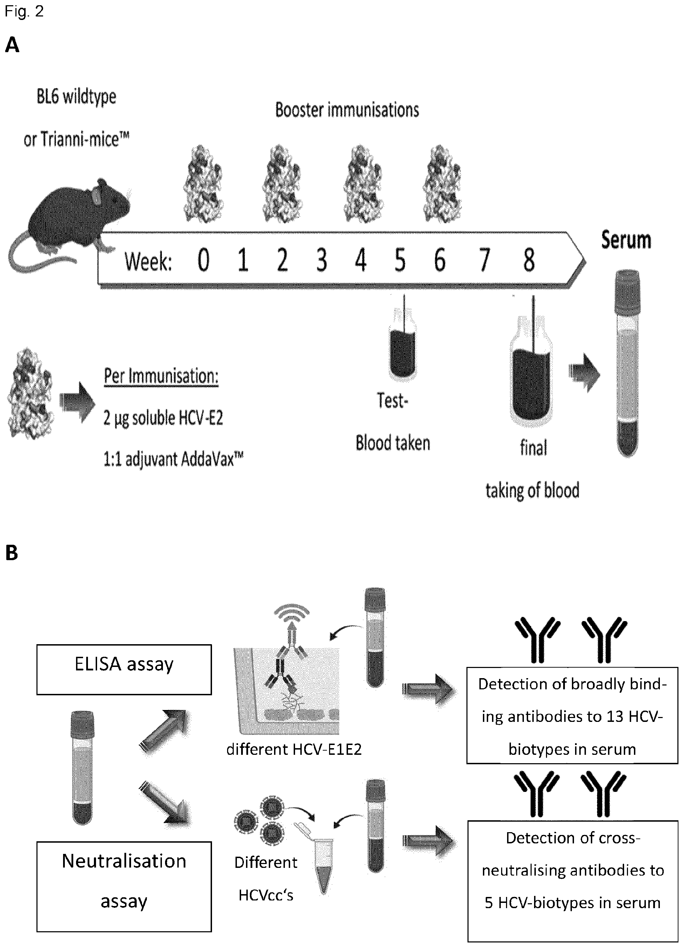

**Fig. 2****: Scheme of the in vitro and in vivo test systems for evaluating the vaccine reaction to soluble HCV E2 in mice.** (A) The in vivo immunisation studies were carried out in C57BI/6 wild type mice as well as in humanised Trianni mice^{™}. The Trianni mice^{™} comprise the complete human repertoire of variable regions of heavy kappa and lambda chains of immunoglobulins, such that, maintaining the mouse specific immunoregulatio, humanized antibodies can be produced. All animals were immunised with 2 µg of soluble HCV E2 protein in a 1:1 mixture with the adjuvant AddaVax^{™} by intramuscular injection at the beginning of the experiment, as well as after 2, 4 and 6 weeks. In week 5 of the experiment, a minimal amount of blood was drawn, and in week 8, the animals were sacrificed and the complete blood volume harvested.The serum was separated by centrifugation and used for following assays. (B) The presence of binding antibodies in serum was tested for selected groups with ELISA assay against the lysates of 13 HCV reference viruses and the presence of neutralising antibodies of all groups was tested by a neutralisation assay against 5 different cell culture generated reference viruses.

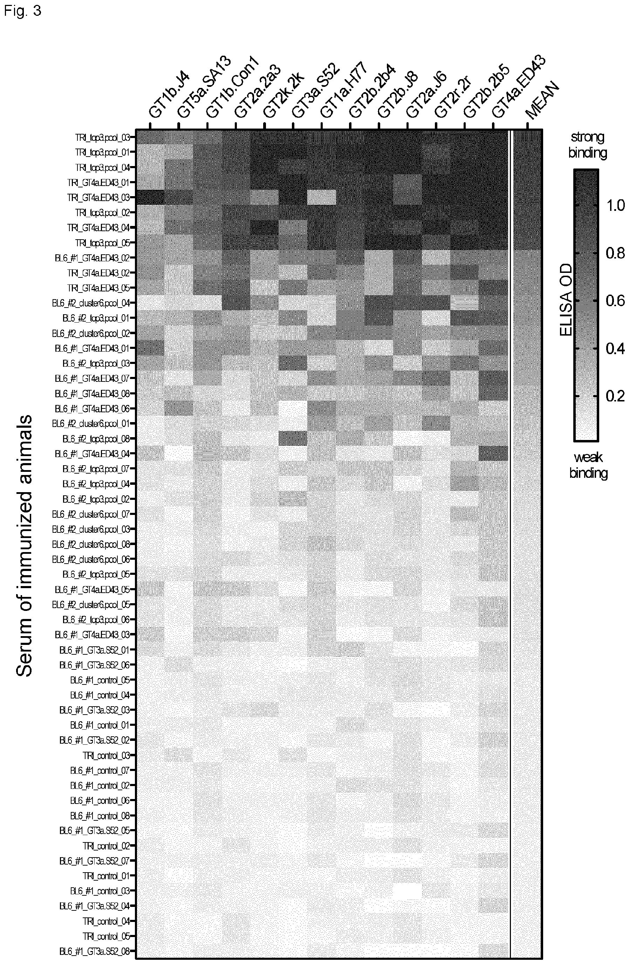

**Fig. 3****: ELISA binding results of sera from immunized BL6 and Trianni^{™} mice against lysates of 13 HCV representatives of the established 6 biotypes.** Ranking of the binding data assessed with the ELISA assay of in total 59 analysed mouse serum samples (lines) against lysates from 13 reference viruses (columns). The OD values are shown in a range between 0 and 1.2 (1.2 being the strongest binding in dark, and 0, the weakest binding, in white).

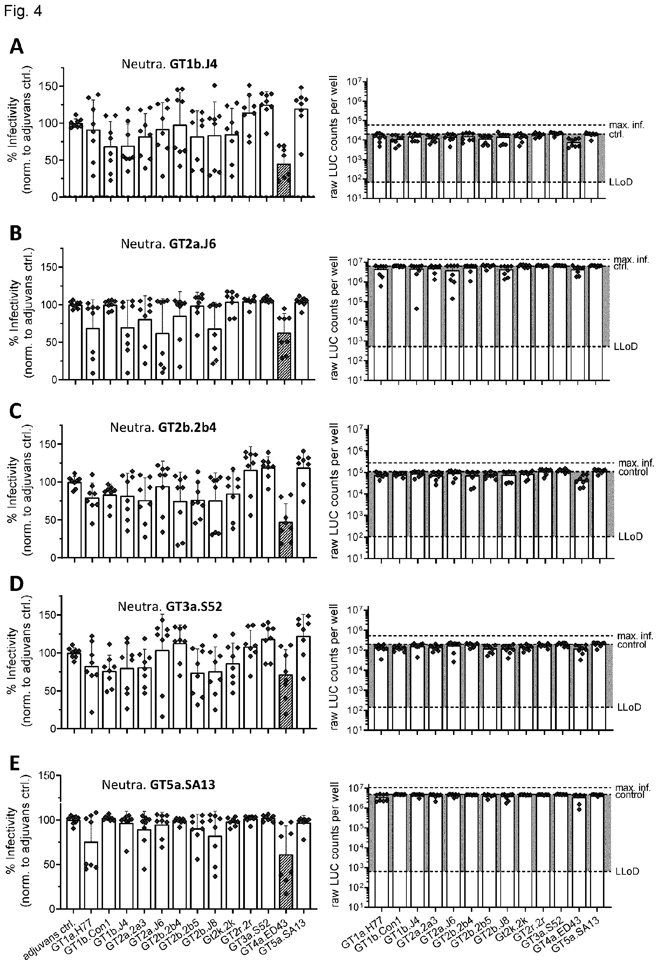

**Fig. 4****: Neutralisation data against reporter viruses from 5 established biotypes and presentation of the dynamic measurement range of the neutralisation assay.** (A-E) Capability for neutralisation of 5 HCV biotypes by a 1:20 dilution of all mouse sera from the BL6 animals immunized with the individual HCV-E2 variants. All data points represent results measured in single animals (n= 8 animals per group), columns represent the average of each group and error bars represent standard deviation (SD). Left side: Representation of the neutralisation of the reference viruses (one reference virus per figure) by the mouse sera as % infectivity normalised to sera of the control animals. Right side: Logarithmic representation of the raw data of the luciferase measurement as an indicator of the remaining infectivity of the reference viruses. Also shown are the maximal possible luciferase activity of all reference viruses ("max. inf."), the reduction in infectivity by addition of the sera of control animals ("ctrl."), the background of the specifc measurement system ("LLoD") and the possible measuring range of the analysis (marked range).

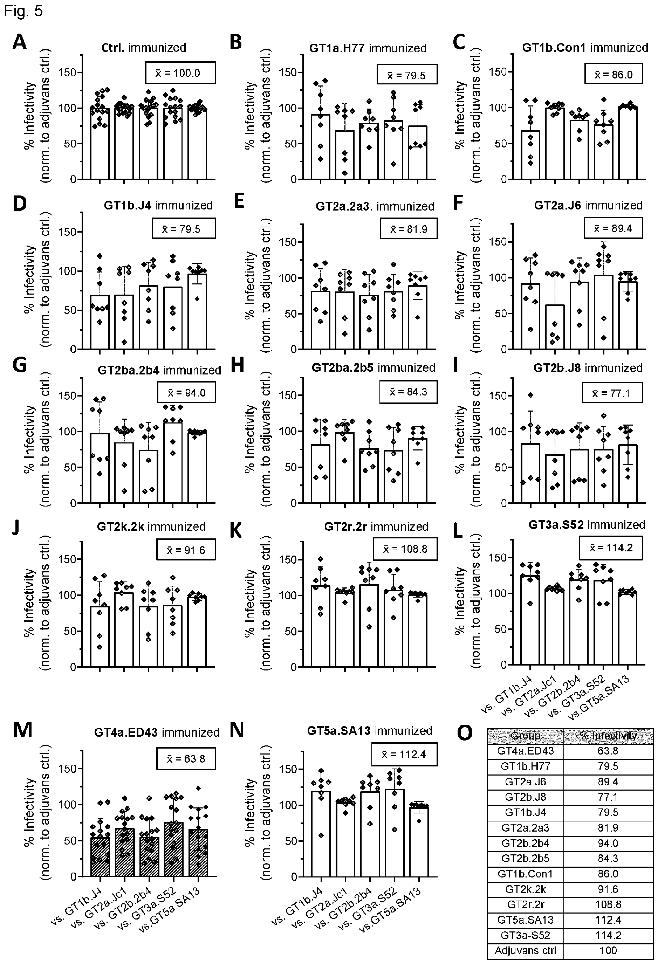

**Fig. 5****: Summary of neutralising data of BL6 mice after individual immunisation with one of 13 HCV-E2 variants each.** (A-N) Neutralisation of the reference viruses by the mouse sera (one group of immunised animals per figure) as % infectivity normalised to sera of the control animals. All data points represent results measured in single animals (n= 8 animals per group), columns represent the average of each group and error bars represent standard deviation (SD). Additionally, the final average (x̅) of the immunized experimental group that leads to neutralisation of all animals of all tested reference viruses and further serves as a central characteristic parameter is specified. (O) Final ranking of the neutralisation efficiencies of the separate groups of immunized mice as ranking of the calculated averages x̅ (shown as % infectivity normalised to sera of the control group).

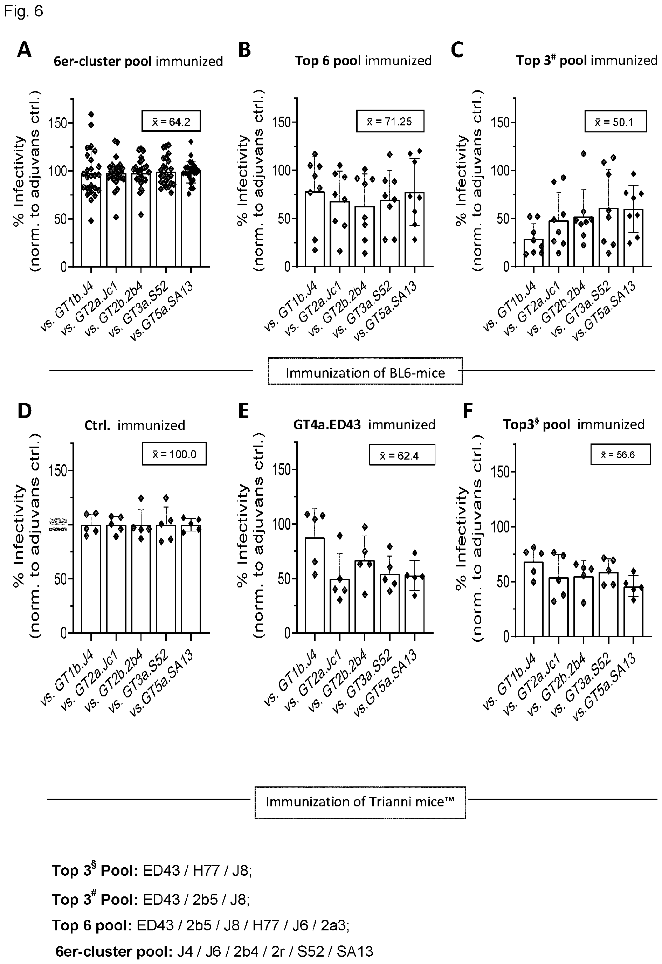

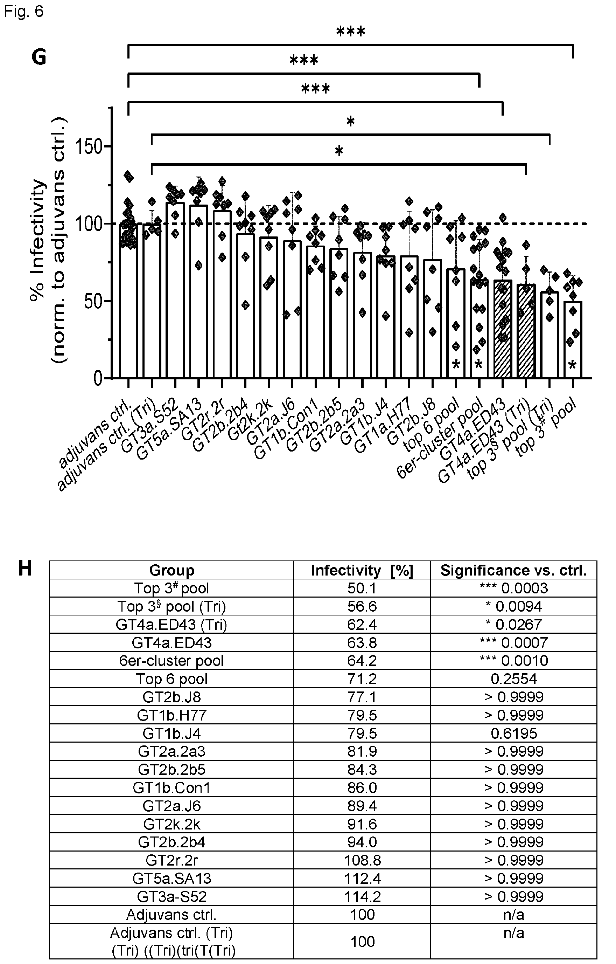

**Fig. 6****: Additional neutralising data of BL6 mice and Trianni^{™} mice after immunisation with different pools of the 13 HCV E2 variants.** (A-) Neutralisation of the reference viruses by the mouse sera (one group of immunised animals per figure) as % infectivity normalized to sera of the control group. A-C: immunisation of BL6 mice. D-F: immunisation of Trianni^{™} mice. (G) Summary of data from Fig. 5 and Fig. 6. Neutralisation data of all reference viruses for the different groups of mice is summarised and serves as a basis for ranking of the candidates. Statistics calculated via Kruskall-Wallis test and Dunn's multiple comparison test (* p < 0.05, **p < 0.005, ** p < 0.0005). For (A-G): All data points represent results measured in single animals (n= 8 animals per group), columns represent the average of each group and error bars represent standard deviation (SD). Additionally, the final average (x̅) of the immunized experimental group that leads to neutralisation of all animals of all tested reference viruses and further serves as a central characteristic parameter is specified. (H) Final ranking of the neutralisation efficiencies of the separate groups of immunized mice as ranking of the calculated averages x̅ (shown as % infectivity normalized to sera of the control group).

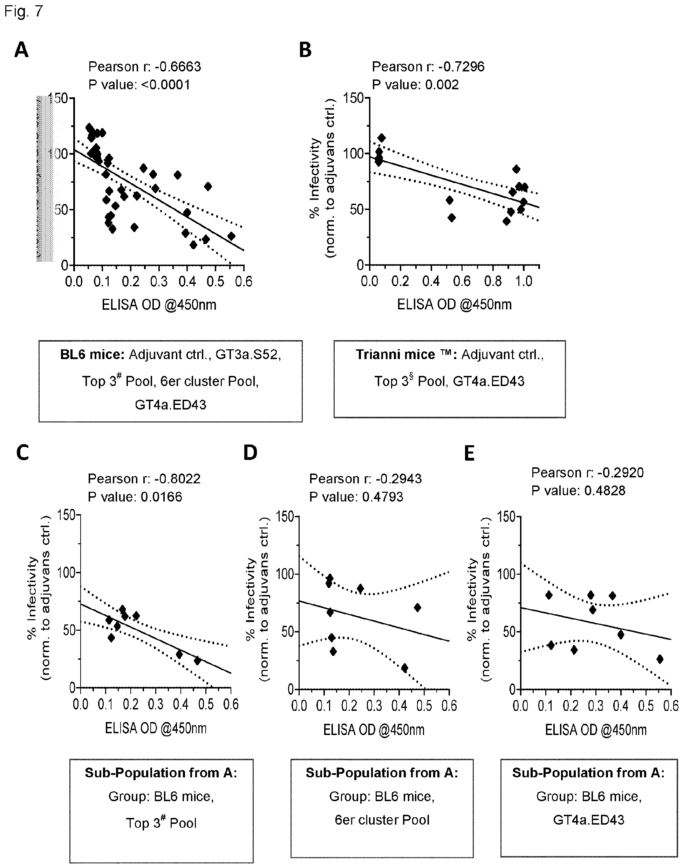

**Fig. 7****: Correlation of binding capacity and neutralisation for specific groups after immunisation with individual or combined HCV E2 variants.** (A-E) Correlation of the binding data (see Fig. 3) with the neutralisation data (see Fig. 5 and 6) for the sera of the immunised mice. (A) Calculation of correlation according to 5 groups of immunised BL6 mice (Adjuvant ctrl., GT3a.S52, Top 3# Pool, 6er cluster Pool, GT4a.ED43). Area between dashed lines: 95% confidence intervall. (B) Calculation of correlation according to 3 groups of immunised Trianni^{™} mice (Adjuvant ctrl., Top 3§ Pool, GT4a.ED43). Area between dashed : 95% confidence intervall. (C-E) Calculation of correlation according to single groups from (A).

### Sequences

SEQ ID NO: 1 sE2 protein of HCV GT4a-ED43 
ETHVSGAAVGRSTAGLANLFSSGSKQNLQLINSNGSWHINRTALNCNDSLNTGFLASL
FYTHKFNSSGCSERLACCKSLDSYGQGWGPLGVANISGSSDDRPYCWHYAPRPCGI
VPASSVCGPVYCFTPSPVVVGTTDHVGVPTYTWGENETDVFLLNSTRPPHGAWFGC
VWMNSTGFTKTCGAPPCEVNTNNGTWHCPTDCFRKHPETTYAKCGSGPWITPRCLID
YPYRLWHFPCTANFSVFNIRTFVGGIEHRMQAACNWTRGEVCGLEHRDRVELSPLLLT
TTAWQILPCSFTTLPALSTGLIHLHQNIVDVQYLYGVGSAVVSWALK
SEQ ID NO: 2 sE2 protein of HCV GT2b.J8 
TTYSSGQEAGRTVLGFTNLFTSGAKQNLYLINTNGSWHINRTALNCNDSLQTGFMASL
FYTHRFNSSGCPERLSSCRGLDDFRIGWGTLEYETHVTNDEDMRPYCWHYPPRPCGI
VPARTVCGPVYCFTPSPVVVGTTDKQGVPTYTWGENETDVFLLNSTRPPRGAWFGC
TWMNGTGFTKTCGAPPCRIRKDYNSTIDLLCPTDCFRKHPDATYLKCGAGPWLTPRC
LVDYPYRLWHYPCTVNFTIFKARMYVGGVEHRFSAACNFTRGDRCRLEDRDRGQQS
PLLHSTTEWAVLPCSFSDLPALSTGLLHLHQNIVDVQYLYGLSPALTRYIVK
SEQ ID NO: 3 sE2 protein of HCV GT2b.2b5 
NTYSSGAQAGRTTSVLTSLFTSGPQQRINLVNTNGSWHINRTALNCNDSLQTGFIASLF
YTHKFNSSGCPERLSSCRTLDDFRIGWGALEYETNVTNDDMRPYCWHYPPKPCGIV
SARTVCGPVYCFTPSPVVVGTTDRQGVPTYSWGENETDVFLLNSTRPPRGAWFGCT
WMNGTGFTKTCGAPPCRIRGDFNATLDLLCPTDCFRKHPDTTYLRCGSGPWLTPRCL
VEYPYRLWHYPCTVNFTISKVRMYVGGVEHRFSAACNFTRGDRCGLEDRDRGQQSP
 LLHSTTEWAVLPCSFSDLPALSTGLLHLHQNIVDVQYLYGLSPALTRYIVK
SEQ ID NO: 4 sE2 protein of HCV GT1a.H77 
ETHVTGGNAGRTTAGLVGLLTPGAKQNIQLINTNGSWHINSTALNCNESLNTGWLAGL
FYQHKFNSSGCPERLASCRRLTDFAQGWGPISYANGSGLDERPYCWHYPPRPCGIVP
AKSVCGPVYCFTPSPVVVGTTDRSGAPTYSWGANDTDVFVLNNTRPPLGNWFGCTW
MNSTGFTKVCGAPPCVIGGVGNNTLLCPTDCFRKHPEATYSRCGSGPWITPRCMVDY
PYRLWHYPCTINYTIFKVRMYVGGVEHRLEAACNWTRGERCDLEDRDRSELSPLLLST
TQWQVLPCSFTTLPALSTGLIHLHQNIVDVQYLYGVGSSIASWAIK
SEQ ID NO: 5 sE2 protein of HCV GT3a.S52 
ETYVTGGSVAHSARGLTSLFSMGAKQKLQLVNTNGSWHINSTALNCNESINTGFIAGL
FYYHKFNSTGCPQRLSSCKPIISFRQGWGPLTDANITGPSDDRPYCWHYAPRPCSVV
PASSVCGPVYCFTPSPVVVGTTDIKGKPTYNWGENETDVFLLESLRPPSGRWFGCAW
MNSTGFLKTCGAPPCNIYGGEGDPENETDLFCPTDCFRKHPEATYSRCGAGPWLTPR
CMVDYPYRLWHYPCTVNFTLFKVRMFVGGFEHRFTAACNWTRGERCNIEDRDRSEQ
HPLLHSTTELAILPCSFTPMPALSTGLIHLHQNIVDVQYLYGVGSDMVGWALK
SEQ ID NO: 6 sE2 protein of HCV GT2r.2r 
HTHVVGGSVAQGARGFTGLFSPGSQQNIQLINTNGSWHINRTALNCNDSLNTGFLASL
FYTHKFNSSGCPERMSACKGIEAFRIGWGVLEHEENVTNDEDMRPYCWHYPPKPCGI
VPARTVCGPVYCFTPSPVVVGTTDRRGVPTYTWGENETDVFLLNSTRPPGGSWFGC
TWMNSTGYTKTCGAPPCRIRADFNASTDLLCPTDCFRKHPDATYGKCGAGPWLTPKC
LVDYPYRLWHYPCTVNFTIFKIRMYVGGVEHRLQAACNFSRGDRCNLEDRDRSQLSP
LLHSTTEWAILPCTFSDMPALSTGLLHLHQNIVDVQYLYGLSPALTKYMVK
SEQ ID NO: 7 sE2 protein of HCV GT2b.2b4 
RTHITGAHVGRTASSLASLFNSGPQQRLSLINTNGSWHINRTALNCNDSLQTGFITALF
YSKHFNSSGCPERLSSCRGLDDFRIGWGPLEYETNVTNGEDMRPYCWHYPPKPCGIV
SAKTVCGPVYCFTPSPVVVGTTDKQGVPTYSWGENETDVFLLNSTRPPQGAWFGCT
WMNGTGYTKTCGAPPCRIRRDYNGTLDLLCPTDCFRKHPDTTYLKCGAGPWLTPKCL
VDYPYRLWHYPCTVNFTIHKVRMYVGGVEHRLNAACNFTRGDRCRLDDRDRGQQSP
LLHSTTEWAVLPCTFSDLPALSTGLLHLHQNIVDVQYLYGLSPAITRYIVK
SEQ ID NO: 8 sE2 protein of HCV GT1b.J4 
ETHTTGRVAGHTTSGFTSLFSSGASQKIQLVNTNGSWHINRTALNCNDSLQTGFFAAL
FYAHKFNSSGCPERMASCRPIDWFAQGWGPITYTKPNSSDQRPYCWHYAPRPCGVV
PASQVCGPVYCFTPSPVVVGTTDRSGVPTYSWGENETDVMLLNNTRPPQGNWFGCT
WMNSTGFTKTCGGPPCNIGGVGNRTLICPTDCFRKHPEATYTKCGSGPWLTPRCLVD
YPYRLWHYPCTLNFSIFKVRMYVGGVEHRLNAACNWTRGERCNLEDRDRSELSPLLL
STTEWQILPCAFTTLPALSTGLIHLHQNIVDVQYLYGVGSAFVSFAIK
SEQ ID NO: 9 sE2 protein of HCV GT5a.SA13 
STRTVGGSAAQGARGLASLFTPGPQQNLQLINTNGSWHINRTALNCNDSLQTGFVAG
LLYYHKFNSTGCPQRMASCRPLAAFDQGWGTISYAAVSGPSDDKPYCWHYPPRPCGI
VPARGVCGPVYCFTPSPVVVGTTDRKGNPTYSWGENETDIFLLNNTRPPTGNWFGCT
WMNSTGFVKTCGAPPCNLGPTGNNSLKCPTDCFRKHPDATYTKCGSGPWLTPRCLV
HYPYRLWHYPCTLNYTIFKVRMYIGGLEHRLEVACNWTRGERCDLEDRDRAELSPLLH
TTTQWAILPCSFTPTPALSTGLIHLHQNIVDTQYLYGLSSSIVSWAVK
SEQ ID NO: 10 sE2 protein of HCV GT2a.J6 
RTHTVGGSAAQTTGRLTSLFDMGPRQKIQLVNTNGSWHINRTALNCNDSLHTGFIASL
FYTHSFNSSGCPERMSACRSIEAFRVGWGALQYEDNVTNPEDMRPYCWHYPPRQC
GVVSAKTVCGPVYCFTPSPVVVGTTDRLGAPTYTWGENETDVFLLNSTRPPLGSWFG
CTWMNSSGYTKTCGAPPCRTRADFNASTDLLCPTDCFRKHPDTTYLKCGSGPWLTP
RCLIDYPYRLWHYPCTVNYTIFKIRMYVGGVEHRLTAACNFTRGDRCNLEDRDRSQLS
 PLLHSTTEWAILPCSYSDLPALSTGLLHLHQNIVDVQFMYGLSPALTKYIVR
SEQ ID NO: 11 sE2 protein of HCV GT2a.2a3 
QTRTIASSVASQASSLVGVFSSGPKQNIQLINTNGSWHINRHWPLTAMTPCNTGFIAAL
VYRNRFNSSGCPERLSACRNIESFRIGWGTLQYENNVTNSEDMRPYCWHYPPKQCDI
VPARSVCGPVYCFTPSPIVVGTTDRLGVPTYTWGENETDVFLLNSTRPPRGSWFGCT
WMNSSGFTKTCGAPPCRIRADFNASTDLLCPTDCFRKHPDATYIKCGSGPWLTPRCL
VDYPYRLWHYPCTVNFTIFKIRMYVGGVEHRLTAACNFTRGDRCNLEDRDRSQLSPLL
HSTTEWAILPCTFSDLPALSTGLLHLHQNIVDVQYMYGLSPALTKYVVR
SEQ ID NO: 12 sE2 protein of HCV GT2k.2k 
QTHTISGHAARTTHGLVSLFTPGSQQNIQLVNTNGSWHINRTALNCNDSLKTGFIAALF
YSHKFNSSGCPQRMSSCRSIEEFRIGWGNLEYEENVTNDDNMRPYCWHYPPRPCGI
VPAQTVCGPVYCFTPSPVVVGTTDRRGVPTYTWGENDTDVFLLNSTRPPRGAWFGC
TWMNSTGFTKTCGAPPCRIRPDFNSSEDLLCPTDCFRKHSEATYTRCGAGPWLTPKC
LFHYPYRLWHYPCTINFTIHKIRMFIGGVEHRLEAACNFTRGDRCNLEDRDRSQLSPLL
HSTTEWAILPCTFSDMPALSTGLLHLHQNIVDVQYLYGLSPAITKYIVK
SEQ ID NO: 13 sE2 protein of HCV 1b.Con1

GTYVTGGTMAKNTLGITSLFSPGSSQKIQLVNTNGSWHINRTALNCNDSLNTGFLAALF
YVHKFNSSGCPERMASCSPIDAFAQGWGPITYNESHSSDQRPYCWHYAPRPCGIVPA
AQVCGPVYCFTPSPVVVGTTDRFGVPTYSWGENETDVLLLNNTRPPQGNWFGCTWM
NSTGFTKTCGGPPCNIGGIGNKTLTCPTDCFRKHPEATYTKCGSGPWLTPRCLVHYPY
RLWHYPCTVNFTIFKVRMYVGGVEHRLEAACNWTRGERCNLEDRDRSELSPLLLSTT
EWQVLPCSFTTLPALSTGLIHLHQNVVDVQYLYGIGSAVVSFAIK

### Examples

### 1. Expression and purification of sE2 proteins

Plasmids encoding individual E2 ectodomains were transfected into Drosophila S2 cells essentially as described previously **(PMID 20174556).** Briefly, stable cell lines were generated by co-transfecting the plasmids coding for the individual E2 ectodomains with a puromycin-resistance selection plasmid. Following selection with puromycin and adaptation of the cells to insect-Xpress media, protein expression was induced with CdCl2 in large scale (2-4L). On day five post-induction, supernatants were harvested, concentrated by tangential flow ultrafiltration and affinity purified from the obtained concentrate using StrepTactin XT Superflow resin, followed by size exclusion chromatography using a Superdex200 column equilibrated in 10mM TRIS pH 8.0, 150mM NaCl. Protein purity assessment was performed by SDS-PAGE analysis.

### 2. Production of infectious cell culture derived Hepatitis C Virus stocks (HCVcc-stocks)

Up to 20 µg of reporter virus plasmid DNA was linearized using an appropriate restriction enzyme. Plasmid DNA was purified using the Qiagen Spin Miniprep kit according to the vendors' instructions. Subsequently, 2µg of restricted and purified plasmid DNA were used as template for *in vitro* transcription. Reactions were completed in a total volume of 100 µL containing the following components: 80 mM HEPES (pH 7.5), 12 mM MgCl₂, 2 mM spermidine, 40 mM dithiothreitol (DTT), a 3.125 mM concentration of each ribonucleoside triphosphate, 1 U RNase inhibitor (Promega), 0.6 U of T7 RNA polymerase (Promega) per µL. After incubation of the reaction mix for 2h at 37°C, 0.3 U T7 polymerase/µL was added and the reaction continued for2 additional hours at 37°C. Subsequently, transcription was terminated by addition of 7.5 U DNAse (Promega) and incubation for 30 minutes at 37°C. *In vitro* transcribed RNA was purified by using the NucleoSpin RNA Clean up kit (Macherey & Nagel) according to manufacturer's instructions. To generate HCVcc reporter virus particles, we electroporated Huh7.5.1 cells with 5µg of *in vitro* transcribed reporter virus RNA. Briefly, single-cell suspensions were prepared by trypsin-treatment and cells were washed with phosphate-buffered saline (PBS), counted and resuspended at a cell density of 10⁷ cells per mL in Cytomix. Cytomix is composed of 2mM ATP, 5mM glutathione, 120 mM KCI , 0.15 mM CaCl₂, 10 mM K₂HPO₄/KH₂PO₄ (pH 7.6); 25 mM Hepes, 2 mM EGTA, 5 mM MgCl₂. ATP and glutathione were added just prior to use. 400µL of the cell suspension in Cytomix were gently mixed with 5 µg in vitro transcribed RNA, transferred to an electroporation cuvette (gap width 0.4 cm; BioRad), and electroporated with a BioRad Gene-pulser using 975 µF, and 270 V settings. Electroporated cells were quickly transferred into fresh DMEM and seeded in cell culture vessels. We collected and pooled supernatants of electroporated cells 48, 72 and 96 h after electroporation, filtered them through a 0.45 µm filter and stored them at - 80°C.

### 3. HCVcc neutralization assay using mouse serum

Neutralization assays were performed as previously described (Bankwitz et al., 2021). In brief, Huh-7.5 cells were seeded at a density of 10⁴ per well in a 96-well plate in 100 µl supplemented DMEM and incubated for 24 h at 37°C and 5% CO₂. Virus strains used herein are 13 different renilla luciferase reporter HCVcc strains (GT1a.H77, GT1b.Con1, GT1b.J4, GT2a.J6, GT2a.2a3, GT2.2b4, GT2b.2b5, GT2b.J8, GT2k.2k, GT2r.2r, GT3a.S52, GT4a.ED43, GT5a.SA13) as previously described in (Bankwitz et al., 2021). HCVcc reporter viruses were mixed with a 1:20 dilution of heat inactivated serum (30 min at 56°C) of immunized mice and incubated for at 37°C for 45 min. This mixture was afterwards used to inoculate cells for 4 h in triplicates at 37°C. Thereafter, 170 µL supplemented DMEM was added onto the cells. Infection was quantified 72 h after virus inoculation by measuring luciferase activity. To this end, cells were washed once with PBS and lysed directly on the plate by addition of 35 µL Milli Q water. After one freeze and thaw cycle, lysates were resuspended and after addition of luciferase substrate (1 µM colenterazine in water) relative light units (RLUs) were measured in a plate luminometer (Lumat LB Centro, Berhold, Germany). The neutralization data was analyzed using GraphPad Prism V9.1.2 (GraphPad Software, La Jolla, California, USA).

### 4. ELISA assay for detection of HCV-binding antibodies

To evaluate the antibody titers of sera from vaccinated mice, nunc-Immuno plates (Thermo Scientific) were coated with cell-lysates of HCV-infected cells. Briefly, Huh-7.5.1 cells were transfected with HCV viral RNA and cell lysates were harvested 96 hours post infection with RIPA buffer. Plates were pretreated with the Galanthus nivalis (GNA) lectin (500 ng/well). Wells were washed twice with TBST (20 mM Tris-HCl pH 7.5, 150 mM NaCl, 0.1% Tween-20, Sigma) and blocked with 100 µl/well of BLOTTO (5% non-fat dry milk, 5% normal goat serum in TBST buffer) and left for 1 h at room temperature. After subsequent washing with TBST, 50 µl of cellular lysate (1:10 dilution in BLOTTO) was used to coat each well of the plate. The mice anti-sera and AP33 monoclonal antibody were diluted in Blotto 1:200. The reaction was developed with the addition of anti-mouse IgG-HRP antibody (sigma 1:1000) and TMB substrate (Sigma) and quenched with 1 M sulfuric acid; absorbance measured at 450 nm (and 630 nm for unspecific background) with an ELISA plate reader (BioTech).

### 5. Comparison and analysis of antibodies induced by different sE2 proteins

The present invention is based on the use of genetically and biologically-functionally different variants of the HCV glycoprotein E2, which can be expressed, purified and adjuvanted as a secreted, truncated protein. It has the following special features and advantages. Protein vaccines have been extensively researched and widely used for a long time (e.g. the annually varying influenza vaccines) and are therefore very well recognized and accepted by the population. In addition, the immunogenicity of the proteins can be varied by the adjuvant and also controlled to a certain extent depending on the choice of adjuvant. However, it was not previously known which HCV envelope proteins or which envelope protein combinations are particularly suitable for a protein-based HCV vaccine candidate.

In our own preliminary work, we have shown that the HCV envelope proteins differ significantly in terms of their biological functions. For example, we have observed that the utilization of cellular entry factors varies between the HCV envelope proteins of different virus types (Haid et al.). Against this background, we formulated the hypothesis that the HCV envelope proteins also differ significantly in terms of their immunogenicity and interaction with human antibodies due to different functional properties. In our large serological study (Bankwitz et al, 2021) we were able to prove that 13 genetically very diverse HCV variants differ significantly in terms of their interaction with human antibodies and can be divided into six different "biotypes" (Fig. 1A and Bankwitz et al , 2021). Representatives of these six biotypes differ significantly in how well they can be neutralized by human antibodies.

Following the assumption that these functional differences (receptor utilization, interaction with antibodies) reflect structural peculiarities of HCV envelope proteins that are also important for immunogenicity and the induction of neutralizing antibodies, we analyzed the immunogenicity of all 13 E2 envelope proteins in an animal model. In addition, we assumed that a combination of these functionally different biotypes could cover a very wide range of different HCV envelope protein types and that, as a combination, a particularly broad immune protection might therefore be built up.

Another important finding of our published study (Bankwitz et al., 2022) was that the genetic sequence of the viruses is not suitable for predicting the biological behavior towards antibodies and that genetically very different viruses can be mapped into a common functional biotype. Based on this, we considered that a combination vaccine grouped by functional biotypes is likely to represent the functional diversity of HCV much better than a combination vaccine that simply accounts for the genetic diversity of the virus. As a result, a combination vaccine composed of functionally diverse envelope proteins should induce a particularly broad protective antibody response that should protect against a broad spectrum of functional viral biotypes.

In the present invention, we have used the full scope of our published study and used the six characterized HCV biotypes with all their 13 representatives (Fig. 1A) to test their potency as a vaccine candidate both individually and in combination of different pools in two different mouse models. The 13 HCV-E2 variants are distributed among the six biotypes as follows:

| Biotype 1 | Biotype 2 | Biotype 3 | Biotype 4 | Biotype 5 | Biotype 6 |
|---|---|---|---|---|---|
| GT1b.Con1 | GT2k.2k | GT2a.2a3 | GT1a.H77 | **GT5a.SA13** | **GT2a.J6** |
| GT2b.2b5 | **GT2r.2r** | **GT2b.2b4** | **GT1b.J4** | | |
| GT2b.J8 | | | GT4a.ED43 | | |
| **GT3a.S52** | | | | | |

### (The representative representatives of the individual biotypes are particularly emphasized)

Expression constructs were cloned from all 13 variants (example in Fig. 1B), the corresponding protein was expressed (as described in Example 1), its quality was checked to ensure quality and to exclude presence of undesired side or degradation products (data not shown) and it was administered to mice in a fixed scheme (Fig. 2A). 2 µg of the corresponding protein were adjuvanted in the injection preparation. This was done by mixing the protein solution 1:1 (volume:volume) with the squalene-based oil-in-water nano-emulsion AddaVax^{™}. This adjuvant is very similar to MF59^{®}, licensed at European level for influenza vaccines. To test our approach, we used two different mouse strains to demonstrate the vectors' immunogenicity in two genetically different models. On the one hand, we used BL6 wild-type animals as a model organism. On the other hand, we replicated selected experimental groups in the humanized Trianni Mice^{™} model (Fig. 2A). Trianni Mice^{™} contain the entire human repertoire of immunoglobulin heavy, kappa and lambda chain variable regions, allowing the production of humanized antibodies while retaining the mouse-specific constant region and regulation. Due to this humanization, the Trianni Mouse^{™} is a particularly predictive model to evaluate the induction of human antibodies by the vaccine candidates.

In accordance with our basic hypothesis, our data show that the different HCV E2 variants screened in the BL6 wild-type mice differ significantly in terms of their immunogenicity. The sera from the immunized animals were tested for HCV glycoprotein-binding antibodies by ELISA assays (as described in Example 4) and for HCV-neutralizing antibodies by neutralization assays (as described in Example 3) (Fig. 2B). The results ranged from test groups whose sera showed neither binding nor neutralizing activity (e.g. GT3a.S52 HCV-E2) to individual candidates (e.g. GT4a.ED43 HCV-E2), but above all combined pools of the individual candidates showed induction of binding and neutralizing antibodies (Fig. 3-7).

In summary, the following important aspects of these vaccine candidates can be derived from the data we have collected from in vitro and especially from in vivo experiments on the mouse model organism: The sera of 5 out of 6 tested immunized experimental groups (4 groups BL6 mice and 2 groups Trianni mice^{™}) indicated the formation of HCV-E2 binding antibodies. This was proven by using HCV-E1E2 ELISA assays (Fig. 3). Overall, the reactivity of the sera of the immunized animals against all homologous 13 E1E2 envelope protein complexes was determined quantitatively in ELISA tests (Fig. 3). These 13 envelope protein complexes are the envelope protein complexes of the reference viruses from the recently published study (Bankwitz et al. 2021). A positive binding (defined as at least 2-fold higher OD value compared to the control group) against at least one E1E2 complex from 13 tested representatives of the 6 biotypes could be detected for each individual from 5 out of 6 tested groups. Accordingly, binding antibodies were successfully induced by a majority, but not all, of the HCV E2 variants (Fig. 3). This finding underscores the importance of screening of a variety of HCV E2 variants to identify suitable candidates.

As the central technology of analysis for estimating the antiviral effect of these antibodies, we used a neutralization assay with which we can detect neutralizing antibodies in the serum of the immunized animals (Bankwitz et al.). Since the neutralization of the antibodies formed by hepatitis C viruses generated in cell culture is the most important parameter, we analyzed the neutralization assay in terms of intemal quality and comparability (Fig. 4) as well as the actual detectability of neutralizing antibodies (Fig. 5 and Fig. 6). Neutralization was tested against five representatives of the published biotypes (GT1b.J4; GT2a.J6; GT2b.2b4; GT3a.S52; GT5a.SA13; Bankwitz et al.) and all biotypes were successfully neutralized by individual mouse sera from both mouse strains used (Fig. 4, left column). In addition, the specific measurement window was defined for the hepatitis C viruses generated in cell culture to be neutralized (Fig. 4, right-hand column, marked area). It was found that the smallest measurement window (GT1b.J4 Figure 4A) can detect a reduction in infectivity up to a factor of 298-fold over the control and the largest measurement window (GT2a.J6 Figure 4B) a reduction in infectivity up to 12,039 -fold over the control. Fig. 5A-N describe the measured neutralization of each group of animals immunized with individual HCV-E2 variants against all tested viruses generated in cell culture. Fig. 5O summarizes these results of each animal group against all tested viruses as an average (x̅) in tabular form. The assays demonstrated no neutralizing activity above background of sera from control animals immunized with the adjuvant AddaVax^{™} alone (Fig. 5A), those with GT3a.S52 (Fig. 5L), GT5a.SA13 (Fig. 5N) or GT2r.2r (Fig. 5K) immunized groups. The remaining groups showed a measurable reduction in % inefctivity over the control animals with residual infectivity ranging from 94.0 % (GT2b.b4; Fig. 5G) to a factor of 63.8 % (GT4a.ED43; Fig. 5M). The latter group thus represents the most promising candidate from the individual immunizations. The adjuvanted immunization with the GT4a.ED43 HCV-E2 most effectively led to the formation of neutralizing antibodies against all five reference viruses tested (Fig. 5M).

Following our basic hypothesis, following the individual testing of the 13 HCV-E2 variants, we put together three different pools and tested them in BL6 wild-type mice (Fig. 6A-C):
Top 3# Pool consisting of: ED43 / 2b5 / J8
Top 6 pool consisting of: ED43 / 2b5 /J8 / H77 /J6 / 2a3
6-cluster pool consisting of: J4 / J6 / 2b4 / 2r / S52 / SA13

As expected, all pools showed induction of neutralizing antibodies in the immunized animals. Surprisingly, the efficiency of the 6-cluster pool (Fig. 6A) was comparable to the best individual candidate GT4a.ED43 (x̅ = 64.2 % vs. x̅ = 63.8 %). However, immunization with top-6pool induced less neutralizing antibodies as compared to GT4a.ED43 (x̅ = 71.2 % vs x̅ = 63.8 %. In conatrast, immunization with the top 3# pool produced a significant increase in neutralization (Figure 6C; x̅ = 50.1 %). In addition, another pool of 3 (ED43 / H77 / J8) and the best individual candidate GT4a.E43 were examined in the humanized Trianni Mice^{™} (Fig. 6D-F). The sera of the immunized Trianni Mice^{™} showed significantly increased ELISA binding titers of the induced HCV-specific antibodies (Fig. 3). However, the neutralization efficiency of the sera was slightly below the corresponding group of BL6 wildtype mice (see summary of all groups and ranking of the mean % Infectivity x̅ in Fig. 6G and 6H).

An additional important parameter is the correlation of the binding properties and the neutralization properties of the antibodies formed by the immunization with the vaccine candidate (Fig. 7). Based on all immunized BL6 wild-type animals for which both ELISA and neutralization assays were performed (n=40), there is a highly significant (P<0.0001) positive correlation between measured binding and measured neutralization in the mouse sera (r = -0.6663) (Fig. 7A). Consideration of the immunized Trianni Mice^{™} (n = 15) revealed a somewhat less pronounced, but nevertheless significant (p = 0.02) positive correlation of r = -0.7296 (Fig. 7B). However, significant differences became visible between the vaccine candidates when the test groups from Fig. 7A were considered separately: Animals immunized with the pool of 3 with the HCV-E2 variants GT4a.ED43, GT2b.2b5 and GT2b.J8 showed the clearest positive correlation with r= 0.8022 (Fig. 7C). The other two experimental groups (Fig. 7D and 7E) showed less clear positive correlations between binding capacity and the ability to neutralize.

### References

Akazawa, D., Moriyama, M., Yokokawa, H., Omi, N., Watanabe, N., Date, T., Morikawa, K., Aizaki, H., Ishii, K., Kato, T., Mochizuki, H., Nakamura, N., Wakita, T., 2013. Neutralizing antibodies induced by cell culture-derived hepatitis C virus protect against infection in mice. Gastroenterology 145, 447-455 e441-444.
Banda, D.H., Perin, P.M., Brown, R.J.P., Todt, D., Solodenko, W., Hoffmeyer, P., Kumar Sahu, K., Houghton, M., Meuleman, P., Muller, R., Kirschning, A., Pietschmann, T., 2019. A central hydrophobic E1 region controls the pH range of hepatitis C virus membrane fusion and susceptibility to fusion inhibitors. J Hepatol 70, 1082-1092.
Bankwitz, D., Bahai, A., Labuhn, M., Doepke, M., Ginkel, C., Khera, T., Todt, D., Stroh, L.J., Dold, L., Klein, F., Klawonn, F., Krey, T., Behrendt, P., Cornberg, M., McHardy, A.C., Pietschmann, T., 2021. Hepatitis C reference viruses highlight potent antibody responses and diverse viral functional interactions with neutralising antibodies. Gut 70, 1734-1745.
Bankwitz, D., Krey, T., Pietschmann, T., 2022. [Development approaches for vaccines against hepatitis C virus infections]. Bundesgesundheitsblatt Gesundheitsforschung Gesundheitsschutz 65, 183-191.
Bankwitz, D., Steinmann, E., Bitzegeio, J., Ciesek, S., Friesland, M., Herrmann, E., Zeisel, M.B., Baumert, T.F., Keck, Z.Y., Foung, S.K., Pecheur, E.I., Pietschmann, T., 2010. Hepatitis C virus hypervariable region 1 modulates receptor interactions, conceals the CD81 binding site, and protects conserved neutralizing epitopes. J Virol 84, 5751-5763.
Beaumont, E., Patient, R., Hourioux, C., Dimier-Poisson, I., Roingeard, P., 2013. Chimeric hepatitis B virus/hepatitis C virus envelope proteins elicit broadly neutralizing antibodies and constitute a potential bivalent prophylactic vaccine. Hepatology 57, 1303-1313.
Capone, S., Zampaglione, I., Vitelli, A., Pezzanera, M., Kierstead, L., Burns, J., Ruggeri, L., Arcuri, M., Cappelletti, M., Meola, A., Ercole, B.B., Tafi, R., Santini, C., Luzzago, A., Fu, T.M., Colloca, S., Ciliberto, G., Cortese, R., Nicosia, A., Fattori, E., Folgori, A., 2006. Modulation of the immune response induced by gene electrotransfer of a hepatitis C virus DNA vaccine in nonhuman primates. J Immunol 177, 7462-7471.
Case, J.B., Rothlauf, P.W., Chen, R.E., Kafai, N.M., Fox, J.M., Smith, B.K., Shrihari, S., McCune, B.T., Harvey, I.B., Keeler, S.P., Bloyet, L.M., Zhao, H., Ma, M., Adams, L.J., Winkler, E.S., Holtzman, M.J., Fremont, D.H., Whelan, S.P.J., Diamond, M.S., 2020. Replication-Competent Vesicular Stomatitis Virus Vaccine Vector Protects against SARS-CoV-2-Mediated Pathogenesis in Mice. Cell Host Microbe 28, 465-474 e464.
Choo, Q.L., Kuo, G., Ralston, R., Weiner, A., Chien, D., Van Nest, G., Han, J., Berger, K., Thudium, K., Kuo, C., et al., 1994. Vaccination of chimpanzees against infection by the hepatitis C virus. Proc Natl Acad Sci U S A 91, 1294-1298.
Christiansen, D., Earnest-Silveira, L., Chua, B., Meuleman, P., Boo, I., Grubor-Bauk, B., Jackson, D.C., Keck, Z.Y., Foung, S.K.H., Drummer, H.E., Gowans, E.J., Torresi, J., 2018. Immunological responses following administration of a genotype 1a/1b/2/3a quadrivalent HCV VLP vaccine. Sci Rep 8, 6483.
Cohen A.A., van Doremalen, N., Greaney A.J., et al. 2022. Mosaic RBD nanoparticles protect against challenge by diverse sarbecoviruses in animal models. Science 10.1126/science.abq0839. Elmowalid, G.A., Qiao, M., Jeong, S.H., Borg, B.B., Baumert, T.F., Sapp, R.K., Hu, Z.Y., Murthy, K., Liang, T.J., 2007. Immunization with hepatitis C virus-like particles results in control of hepatitis C virus infection in chimpanzees. P Natl Acad Sci USA 104, 8427-8432.
Ezelle, H.J., Markovic, D., Barber, G.N., 2002. Generation of hepatitis C virus-like particles by use of a recombinant vesicular stomatitis virus vector. J Virol 76, 12325-12334.
Frey, S.E., Houghton, M., Coates, S., Abrignani, S., Chien, D., Rosa, D., Pileri, P., Ray, R., Di Bisceglie, A.M., Rinella, P., Hill, H., Wolff, M.C., Schultze, V., Han, J.H., Scharschmidt, B., Belshe, R.B., 2010. Safety and immunogenicity of HCV E1E2 vaccine adjuvanted with MF59 administered to healthy adults. Vaccine 28, 6367-6373.
Gottwein, J.M., Bukh, J., 2013. Viral hepatitis: Cell-culture-derived HCV--a promising vaccine antigen. Nat Rev Gastroenterol Hepatol 10, 508-509.
Gottwein, J.M., Scheel, T.K.H., Jensen, T.B., Lademann, J.B., Prentoe, J.C., Knudsen, M.L., Hoegh, A.M., Bukh, J., 2009. Development and Characterization of Hepatitis C Virus Genotype 1-7 Cell Culture Systems: Role of CD81 and Scavenger Receptor Class B Type I and Effect of Antiviral Drugs. Hepatology 49, 364-377.
Haid, S., Grethe, C., Dill, M.T., Heim, M., Kaderali, L., Pietschmann, T., 2014. Isolate-dependent use of claudins for cell entry by hepatitis C virus. Hepatology 59, 24-34.
Hills, R.A., Tan, T.K., Cohen, A.A., et al., 2023. Multiviral Quartet Nanocages Elicit Broad Anti-Coronavirus Responses for Proactive Vaccinology. bioRxiv preprint, https://doi.org/10.1101/2023.02.24.529520
Hoffmann M.A.G., Yang, Z., Huey-Tibman K.E., et al., 2023, ESCRT recruitment to SARS-CoV-2 spike induces virus-like particles that improve mRNA vaccines. Cell 186, 2380-2391
Jones, S.M., Feldmann, H., Stroher, U., Geisbert, J.B., Fernando, L., Grolla, A., Klenk, H.D., Sullivan, N.J., Volchkov, V.E., Fritz, E.A., Daddario, K.M., Hensley, L.E., Jahrling, P.B., Geisbert, T.W., 2005. Live attenuated recombinant vaccine protects nonhuman primates against Ebola and Marburg viruses. Nat Med 11, 786-790.
Kinchen, V.J., Zahid, M.N., Flyak, A.I., Soliman, M.G., Learn, G.H., Wang, S., Davidson, E., Doranz, B.J., Ray, S.C., Cox, A.L., Crowe, J.E., Jr., Bjorkman, P.J., Shaw, G.M., Bailey, J.R., 2018. Broadly Neutralizing Antibody Mediated Clearance of Human Hepatitis C Virus Infection. Cell Host Microbe 24, 717-730 e715.
Law, M., Maruyama, T., Lewis, J., Giang, E., Tarr, A.W., Stamataki, Z., Gastaminza, P., Chisari, F.V., Jones, I.M., Fox, R.I., Ball, J.K., McKeating, J.A., Kneteman, N.M., Burton, D.R., 2008. Broadly neutralizing antibodies protect against hepatitis C virus quasispecies challenge. Nat Med 14, 25-27. Nestola, P., Peixoto, C., Silva, R.R., Alves, P.M., Mota, J.P., Carrondo, M.J., 2015. Improved virus purification processes for vaccines and gene therapy. Biotechnol Bioeng 112, 843-857.
Osburn, W.O., Fisher, B.E., Dowd, K.A., Urban, G., Liu, L., Ray, S.C., Thomas, D.L., Cox, A.L., 2010. Spontaneous control of primary hepatitis C virus infection and immunity against persistent reinfection. Gastroenterology 138, 315-324.
Osburn, W.O., Snider, A.E., Wells, B.L., Latanich, R., Bailey, J.R., Thomas, D.L., Cox, A.L., Ray, S.C., 2014. Clearance of hepatitis C infection is associated with the early appearance of broad neutralizing antibody responses. Hepatology 59, 2140-2151.
Pestka, J.M., Zeisel, M.B., Blaser, E., Schurmann, P., Bartosch, B., Cosset, F.L., Patel, A.H., Meisel, H., Baumert, J., Viazov, S., Rispeter, K., Blum, H.E., Roggendorf, M., Baumert, T.F., 2007. Rapid induction of virus-neutralizing antibodies and viral clearance in a single-source outbreak of hepatitis C. Proc Natl Acad Sci U S A 104, 6025-6030.
Ray, R., Meyer, K., Banerjee, A., Basu, A., Coates, S., Abrignani, S., Houghton, M., Frey, S.E., Belshe, R.B., 2010. Characterization of antibodies induced by vaccination with hepatitis C virus envelope glycoproteins. J Infect Dis 202, 862-866.
Sliepen, K., Radic, L., Capella-Pujol, J., et al., Induction of cross-eutralizing antibodies y a permutated hepatitis C virus glycoprotein nanoparticle vaccine candidate. Nature Communications 13:7271.
Smith, D.B., Bukh, J., Kuiken, C., Muerhoff, A.S., Rice, C.M., Stapleton, J.T., Simmonds, P., 2014. Expanded classification of hepatitis C virus into 7 genotypes and 67 subtypes: updated criteria and genotype assignment web resource. Hepatology 59, 318-327.
Sourisseau, M., Michta, M.L., Zony, C., Israelow, B., Hopcraft, S.E., Narbus, C.M., Parra Martin, A., Evans, M.J., 2013. Temporal analysis of hepatitis C virus cell entry with occludin directed blocking antibodies. PLoS Pathog 9, e1003244.
Swadling, L., Capone, S., Antrobus, R.D., Brown, A., Richardson, R., Newell, E.W., Halliday, J., Kelly, C., Bowen, D., Fergusson, J., Kurioka, A., Ammendola, V., Del Sorbo, M., Grazioli, F., Esposito, M.L., Siani, L., Traboni, C., Hill, A., Colloca, S., Davis, M., Nicosia, A., Cortese, R., Folgori, A., Klenerman, P., Barnes, E., 2014. A human vaccine strategy based on chimpanzee adenoviral and MVA vectors that primes, boosts, and sustains functional HCV-specific T cell memory. Sci Transl Med 6, 261ra153.
Tarr, A.W., Urbanowicz, R.A., Hamed, M.R., Albecka, A., McClure, C.P., Brown, R.J., Irving, W.L., Dubuisson, J., Ball, J.K., 2011. Hepatitis C patient-derived glycoproteins exhibit marked differences in susceptibility to serum neutralizing antibodies: genetic subtype defines antigenic but not neutralization serotype. J Virol 85, 4246-4257.
Urbanowicz, R.A., McClure, C.P., Brown, R.J., Tsoleridis, T., Persson, M.A., Krey, T., Irving, W.L., Ball, J.K., Tarr, A.W., 2015. A Diverse Panel of Hepatitis C Virus Glycoproteins for Use in Vaccine Research Reveals Extremes of Monoclonal Antibody Neutralization Resistance. J Virol 90, 3288-3301.
Vanwolleghem, T., Bukh, J., Meuleman, P., Desombere, I., Meunier, J.C., Alter, H., Purcell, R.H., Leroux-Roels, G., 2008. Polyclonal immunoglobulins from a chronic hepatitis C virus patient protect human liver-chimeric mice from infection with a homologous hepatitis C virus strain. Hepatology 47, 1846-1855.
Wang, R., Suzuki, S., Guest, J.D., Heller, B., Almeda, M., Andrianov, A.K., Marin, A., Mariuzza, R.A., Keck, Z.Y., Foung, S.K.H., Yunus, A.S., Pierce, B.G., Toth, E.A., Ploss, A., Fuerst, T.R., 2022. Induction of broadly neutralizing antibodies using a secreted form of the hepatitis C virus E1E2 heterodimer as a vaccine candidate. Proc Natl Acad Sci U S A 119, e2112008119.
Wasilewski, L.N., Ray, S.C., Bailey, J.R., 2016. Hepatitis C virus resistance to broadly neutralizing antibodies measured using replication-competent virus and pseudoparticles. J Gen Virol 97, 2883-2893.
Weber, T., Potthoff, J., Bizu, S., Labuhn, M., Dold, L., Schoofs, T., Horning, M., Ercanoglu, M.S., Kreer, C., Gieselmann, L., Vanshylla, K., Langhans, B., Janicki, H., Stroh, L.J., Knops, E., Nierhoff,
D., Spengler, U., Kaiser, R., Bjorkman, P.J., Krey, T., Bankwitz, D., Pfeifer, N., Pietschmann, T., Flyak, A.I., Klein, F., 2022. Analysis of antibodies from HCV elite neutralizers identifies genetic determinants of broad neutralization. Immunity 55, 341-354 e347.
Wolf, M.W., Reichl, U., 2011. Downstream processing of cell culture-derived virus particles. Expert Rev Vaccines 10, 1451-1475.
Yokokawa, H., Higashino, A., Suzuki, S., et al., 2018. Induction of humoural and cellular immunity by immunisation with HCV particle vaccine in a non-human primate model. Gut 67, 372-379.
Zheng, A., Yuan, F., Li, Y., Zhu, F., Hou, P., Li, J., Song, X., Ding, M., Deng, H., 2007. Claudin-6 and claudin-9 function as additional coreceptors for hepatitis C virus. J Virol 81, 12465-12471.

## Claims

1. A composition comprising glycoprotein E2 from HCV strain GT4a.ED43, wherein the glycoprotein is
a) a soluble glycoprotein not comprising a transmembrane region, and/or
b) displayed on the surface of a nanoparticle,
optionally, wherein the composition comprises an adjuvant.

2. A composition of claim 1 comprising glycoprotein E2 from HCV strain GT4a.ED43 having at least 80% sequence identity to SEQ ID NO: 1.

3. The composition of any of claims 1 or 2 comprising the soluble glycoprotein and an adjuvant.

4. The composition of any of claims 1 or 2, comprising the glycoprotein displayed on the surface of a nanoparticle.

5. A composition comprising a nucleic acid encoding glycoprotein E2 from HCV strain GT4a.ED43 having at least 80% sequence identity to SEQ ID NO: 1, wherein the nucleic acid is
a) RNA, optionally, mRNA formulated in a lipid nanoparticle, or
b) a non-HCV viral nucleic acid, optionally, comprised in a viral vecor selected from the group comprising a VSV vector, a rLCMV vector, an adenovirus vector, an MVA vector and an RNA replicon vector,
c) DNA, wherein the nucleic acid encoding the soluble E2 glycoprotein preferably is operatively linked to a heterologous promotor capable of mediating expression in human cells,
wherein, preferably, the nucleic acid is RNA.

6. The composition of any of the preceding claims, wherein said glycoprotein E2 does not comprise the 31 C-terminal amino acids of wild-type E2 glycoprotein of said strain.

7. The composition of any of the preceding claims, wherein said glycoprotein E2 has at least 90% amino acid identity to SEQ ID NO: 1, wherein, preferably, the sequence of the glycoprotein E2 comprises SEQ ID NO: 1.

8. The composition of any of the preceding claims, further comprising HCV glycoprotein E2 from HCV strain GT2b.J8, or a nucleic acid encoding said HCV glycoprotein E2, wherein the nucleic acid is a) RNA, b) a non-HCV viral nucleic acid, or c) DNA, wherein the nucleic acid encoding the E2 glycoprotein preferably is operatively linked to a heterologous promotor capable of mediating expression in human cells,
wherein said glycoprotein E2 from HCV strain GT2b.J8 has at least 90% amino acid identity to SEQ ID NO: 2.

9. The composition of any of the preceding claims, further comprising HCV glycoprotein E2 from HCV strains GT2b.2b5 and GT2b.J8, or a nucleic acid encoding said HCV glycoprotein E2, wherein the nucleic acid is a) RNA, b) a non-HCV viral nucleic acid, or c) DNA, wherein the nucleic acid encoding the E2 glycoprotein preferably is operatively linked to a heterologous promotor capable of mediating expression in human cells,
wherein said glycoprotein E2 from HCV strain GT2b.2b5 has at least 90% amino acid identity to SEQ ID NO: 3.

10. The composition of any of the preceding claims, further comprising HCV glycoprotein E2 from HCV strains GT1a.H77 and GT2b.J8, or a nucleic acid encoding said HCV glycoprotein E2, wherein the nucleic acid is a) RNA, b) a non-HCV viral nucleic acid, or c) DNA, wherein the nucleic acid encoding the E2 glycoprotein preferably is operatively linked to a heterologous promotor capable of mediating expression in human cells,
wherein said glycoprotein E2 from HCV strain GT1a.H77 has at least 90% amino acid identity to SEQ ID NO: 4.

11. The composition of any of the preceding claims comprising an adjuvant, wherein the adjuvant optionally comprises a squalen-based oil in water nano-emulsion.

12. The composition of any of the preceding claims, further comprising a HCV E1 protein, optionally, a soluble HCV E2 protein, if the composition comprises the E2 protein, or nucleic acid encoding a HCV E1 protein, optionally, a soluble HCV E2 protein, if the composition comprises a nucleic acid encoding the E2 protein, wherein preferably the E1 and the E2 protein are from the same strain.

13. The composition of any of the preceding claims, comprising at least one further HCV protein or a nucleic acid encoding the same, wherein the further HCV protein is selected from the group consisting of P7, N2, NS3, NS4A, NS4B, NS5A and NS5B.

14. A vaccine comprising the composition of any of the preceding claims, optionally, in combination with a vaccine against another infectious agent selected from the group comprising hepatitis B, hepatitis A, hepatitis E, pertussis, diphteria, tetanus, poliomyelitis, haemophilus B, measles, influenza,, SARS-CoV-2, FSME, or HPV.

15. The vaccine of claim 14 for use in prophylactic vaccination of a subject against HCV, optionally, a subject that has successfully been treated for a chronic HCV infection.

16. The vaccine for use of claim 15, wheren said vaccine is administered in a prime boost regimen, wherein it is used for priming and/or for boosting an immune response to HCV, preferably, for both.

17. A method for producing the composition of any of claims 1-4 or 6-13 or the vaccine of claim 14 or the vaccine for use of any of claims 15 or 16, comprising
a) culturing a cell expressing the soluble gylcoprotein E2, and
b) isolating said glycoprotein, and
c) optionally, formulating said glycoprotein in a formulation suitable for storage and/or administration as a vaccine,
or a method for producing the composition of any of claims 5-13 or the vaccine of claim 14 or the vaccine for use of any of claims 15 or 16, wherein the nucleic acid is an mRNA, the method comprising
i) preparing said nuleic acid, optionally, comprising culturing a cell expressing the nucleic acid, and isolating said nucleic acid, and
ii) packaging said nucleic acid in a lipid nanoparticle and
iii) optionally, formulating said lipid nanoparticle in a formulation suitable for storage and/or administration as a vaccine,
or a method for producing the composition of any of claims 5-13 or the vaccine of claim 14 or the vaccine for use of any of claims 15 or 16, wherein the nucleic acid is a viral nucleic acid, the method comprising
I. comprising culturing a cell comprising the nucleic acid, and
II. packaging said nucleic acid in the viral vector and
III. optionally, formulating said lipid vector in a formulation suitable for storage and/or administration as a vaccine.
